# EUROPÄISCHE PATENTSCHRIFT

(11) **EP 0 306 443 B1**
(45) Veröffentlichungstag und Bekanntmachung des Hinweises auf die Patenterteilung: **01.12.1993**
(21) Anmeldenummer: 88730201.6
(22) Anmeldetag: 05.09.1988
(51) Int. Cl.: A61N 1/36, A61N 1/375, A61N 1/05

(54) **Koaxialstecker für Herzschrittmacher**
Coaxial connector for a heart pace-maker
Prise coaxiale pour stimulateur cardiaque

(30) Priorität: 03.09.1987 DE 8712042 U
(43) Veröffentlichungstag der Anmeldung: 08.03.1989
(73) Patentinhaber: BIOTRONIK Mess- und Therapiegeräte GmbH & Co Ingenieurbüro Berlin, D-12359 Berlin (DE)
(72) Erfinder: Kormann, Décio, S., Paraiso, Sao Paulo (BR)
(74) Vertreter: Christiansen, Henning, Dipl.-Ing.

(56) Entgegenhaltungen:
- EP-A- 0 052 879
- DE-A- 3 338 838
- US-A- 4 236 525

## Beschreibung

Die Erfindung betrifft einen Koaxialstecker der im Oberbegriff des Anspruchs 1 angegebenen Art.

Zur Verbindung von unipolaren oder bipolaren Herzschrittmachern mit einer oder zwei im Herz eines Patienten zu plazierenden Elektroden dienen Elektrodenleiter oder -kabel, an deren einem Ende ein Elektrodenkopf mit einem elektrisch leitfähigen Bereich zur Übertragung der Schrittmacherimpulse auf den Myokard und an deren anderem Ende ein Stecker vorgesehen ist, der in eine entsprechende Steckbuchse im Schrittmachergehäuse eingefügt wird.

Bei unipolaren Herzschrittmachern bildet das Metallgehäuse des Schrittmachers eine indifferente Elektrode aus, die das Bezugspotential für die Stimulationsimpulse bildet. Zur Verbindung mit der einen, im Herz eines Patienten zu implantierenden Elektrode ist daher nur ein einzelner Stecker erforderlich, der in einfacher Weise in einer entsprechenden Steckbuchse des Schrittmachergehäuses einfügbar ist.

Bei der bipolaren Stimulationsart waren jedoch zur Verbindung mit einer Anoden- und Kathodenelektrode über entsprechende Anoden- und Kathodenleiter zwei Stecker erforderlich, was sowohl einen mechanischen als auch einen kosmetisch störenden Faktor darstellt. Da die bipolare Stimulation gegenüber einer unipolaren Stimulationsart aber den Vorteil bietet, daß eine Beeinflussung des Sensing-Verhaltens durch externe Störfelder praktisch ausgeschlossen ist, während bei der unipolaren Stimulationsform durch den großen räumlichen Abstand zwischen Kathode und Anode die Elektrode als Antenne wirkt, ist man bestrebt, die bei der bipolaren Stimulationsart erforderlichen zwei Stecker durch einen einzelnen Stecker zu ersetzen.

Aus der EP 0 052 879 B1 ist ein bipolarer Koaxialstecker zum Verbinden des proximalen Endes eines implantierbaren Elektrodenleiters mit einem implantierbaren Herzschrittmacher bekannt, der eine Bohrung zur Aufnahme des proximalen Endes des Elektrodenleiters und mehrere elektrisch leitende Flächen aufweist, die durch elektrisch isolierende Dichtungselemente voneinander getrennt sind.

Aus US 4 236 525 ist ein Koaxialstecker nach dem Oberbegriff des Anspruchs 1 bekannt.

Zum Plazieren der Elektrode bei einem unipolaren Herzschrittmacher oder der Elektroden bei einem bipolaren Herzschrittmacher verwendet man einen Führungsdraht, den sog. Mandrin, der durch ein Lumen des Elektrodenleiters bis zum Elektrodenkopf gesteckt wird und durch Dreh- und Vorschubbewegungen den Elektrodenkopf an einer geeigneten Stelle plaziert und ihn dort gegebenenfalls durch Drehen des Mandrins verankert. Aus diesem Grund sind einerseits geeignete Elektrodenleiter oder -kabel erforderlich, die ein entsprechendes Lumen aufweisen und andererseits Stecker, die ein Einführen des Mandrins sowie eine leichte Handhabung beim Plazieren des Elektrodenkopfes ermöglichen.

Beide genannten Druckschriften beschreiben keinen solchen Stecker.

Der im Anspruch 1 angegebenen Erfindung liegt daher die Aufgabe zugrunde, einen Koaxialstecker zu schaffen, der bei einfacher Handhabung die Einführung eines Mandrin sowohl zum Plazieren der Anode als auch der Kathode gestattet und der eine körperflüssigkeitsdichte Verbindung mit einer entsprechenden Steckbuchse im Schrittmachergehäuse gewährleistet.

Diese Aufgabe wird durch den Koaxialstecker gemäß Anspruch 1 gelöst. Die erfindungsgemäße Lösung schafft einen Koaxialstecker, der die Verbindung eines getrennten Anoden- und Kathodenleiters und das getrennte Plazieren der Anode und Kathode ermöglicht, eine einfache Handhabung beim Plazieren mit Hilfe eines Mandrins gewährleistet und sicherstellt, daß eine körperflüssigkeitsdichte Verbindung mit einer Steckbuchse eines Schrittmachergehäuses und ein sicherer elektrischer Kontakt mit den entsprechenden Polen des Schrittmachers gegeben ist.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 eine teilweise geschnittene Darstellung des Koaxialsteckers und der Anoden und Kathodenelektrode;
Figur 2 einen Schnitt durch den Koaxialstecker entlang der Linie A - A gemäß Figur 1;
Figur 3 eine Draufsicht auf den Koaxialstecker gemäß Figur 1;
Figur 4 eine Seitenansicht des Koaxialsteckers gemäß Figur 1 und
Figur 5 eine teilweise geschnittene Darstellung des bipolaren Herzschrittmachers.

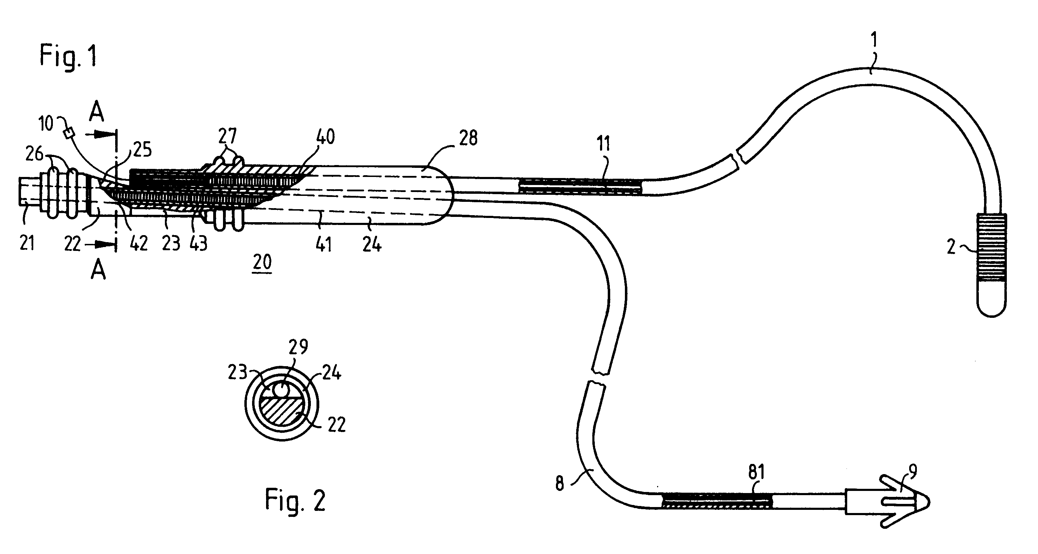

Das in Figur 1 dargestellte Ausführungsbeispiel eines erfindungsgemäßen Koaxialsteckers 20 zeigt die Verbindung des Koaxialsteckers 20 mit einem Anodenleiter 1 und einem Kathodenleiter 8, die getrennt aus der Stirnseite des Steckergehäuses 28 herausgeführt sind und an ihren dem Koaxialsteckcker 20 entgegengesetzten Enden eine Anode 2 bzw. eine Kathode 9 aufweisen.

Der Anodenleiter 1 sowie Kathodenleiter 8 sind vorzugsweise als mehrwendelige Elektrodenleiter ausgebildet, die ein Lumen 11 bzw. 81 ausbilden, in dem ein in Figur 1 schematisch dargestellter Mandrin 10 zum Plazieren der Anode 2 bzw. Kathode 9 einführbar ist. In dem dargestellten Ausführungsbeispiel besteht die Anode 2 aus einer zylindrisch ausgebildeten Elektrode mit einem wendel- oder spiralförmig gestreckten Leiter, die vorzugsweise im Bereich des Brustbeins eines Patienten zu plazieren ist. Die Kathode ist in ihrer Spitze halbkugelförmig ausgebildet und weist mehrere sog. Schaldach-Widerhaken zur aktiven Fixation auf.

Der Koaxialstecker 20 enthält einen ersten zylindrischen Abschnitt 21, an dessen Stirnseitenende wahlweise ein Steckkontakt oder eine Buchse vorgesehen ist, der bzw. die mit dem Kathodenleiter 8 und einer entsprechenden Buchse oder einem Stecker im bipolaren Herzschrittmacher elektrisch verbindbar sind. An seinem äußeren Umfang ist der erste zylindrische Abschnitt 21 mit zwei Dichtringen 26 versehen, die in entsprechende Dichtnuten im Schrittmachergehäuse einrasten, wenn der Koaxialstecker 20 vollständig in das Schrittmachergahäuse eingesteckt ist.

An den ersten zylindrischen Abschnitt 21 schließt ein im Durchmesser geringfügig größerer zweiter zylindrischer Abschnitt 22 an, der eine Ausnehmung 25 aufweist, die im dargestellten Ausführungsbeispiel aus einer schrägen, ebenen Fläche besteht, die mit der Stirnseite des anschliessenden dritten zylindrischen Abschnittes 23 einen stumpfen Winkel bildet.

Der dritte zylindrische Abschnitt 23 weist den gleichen Durchmesser auf wie der zweite zylindrische Abschnitt 22 und enthält an seiner der Ausnehmung 25 zugewandten Stirnseite eine Mandrinbohrung 29 (Figur 2) und ist an seiner Oberfläche mit einer elektrisch leitenden Kontaktfläche versehen, die mit dem Anodenleiter 1 verbunden ist und als positiver oder Anodenkontakt zur Verbindung mit einer entsprechenden Buchse im Innern des Herzschrittmachers dient. Die Kontaktfläche kann bis zum zweiten zylindrischen Abschnitt 22 reichen oder kürzer ausgebildet sein.

Ein an den dritten zylindrischen Abschnitt 23 anschließender vierter zylindrischer Abschnitt 24 weist einen gegenüber dem zweiten und dritten zylindrischen Abschnitt 22, 23 etwas vergrößerten Durchmesser auf und ist analog zum ersten zylindrischen Abschnitt 21 an seinem dem dritten zylindrischen Abschnitt 23 benachbarten Ende mit zwei Dichtringen 27 versehen, die in entsprechende Dichtnuten im Schrittmachergehäuse bei vollständig eingestecktem Koaxialstecker einrasten. Aus der Stirnseite 28 des vierten zylindrischen Abschnittes 24 treten getrennt voneinander der Anoden- und Kathodenleiter 1 bzw. 8 aus.

Der vierte zylindrische Abschnitt 24 enthält eine erste axial durchgehende Bohrung 40, die mit der Mandrinbohrung 29 im dritten zylindrischen Abschnitt fluchtet und schräg verläuft, so daß die seitlich austretende Bohrung 29 mit einem mittigen Austritt an der Stirnseite 28 des vierten zylindrischen Abschnitts 24 verbunden ist.

Eine zweite axial durchgehende Bohrung 41 im zylindrischen Abschnitt 24 fluchtet mit zwei im zweiten und dritten zylindrischen Abschnitt 22, 23 vorgesehenen Bohrungen 42,43 sowie dem Steckkontakt bzw. der Steckbuchse im ersten zylindrischen Abschnitt 21 derart, daß ein Mandrin in eine Bohrung des Steckkontaktes bzw. der Steckbuchse einführbar und in die durchgehende Bohrung 32, 33, 41 eingesteckte Kathodenleitung einführbar ist.

Figur 2 verdeutlicht die Lage der Mandrinbohrung 29 im dritten zylindrischen Abschnitt 23.

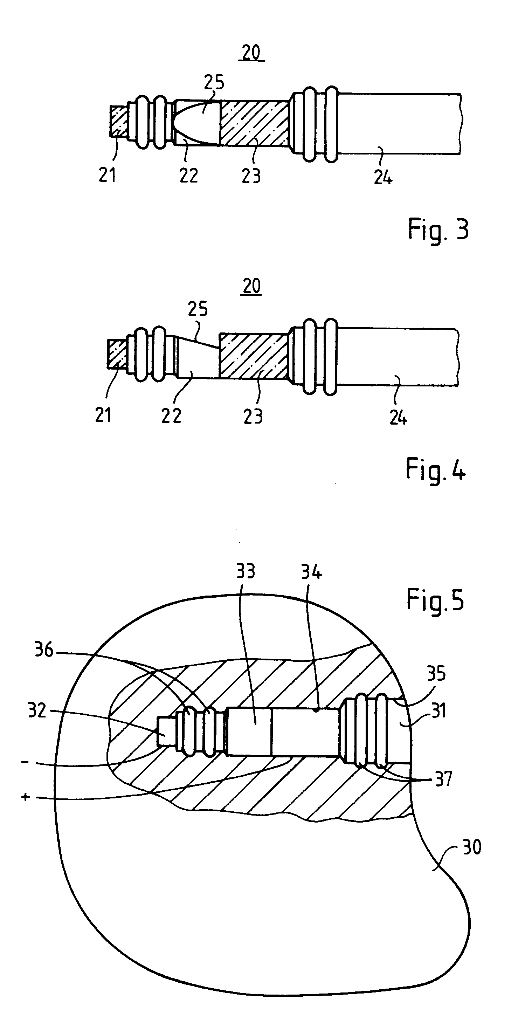

Die Figuren 3 und 4 verdeutlichen in Draufsicht bzw. in Seitenansicht des Koaxialsteckers 20 die Lage und Ausbildung der Ausnehmung im zweiten zylindrischen Abschnitt 22.

Figur 5 zeigt einen im Bereich einer Steckbuchse 31 zur Aufnahme des Koaxialsteckers 20 geschnittenen bipolaren Herzschrittmacher 30.

Die Steckbuchse 31 im bipolaren Herzschrittmacher 30 weist einen ersten hohlzylindrischen Abschnitt 32 zur Aufnahme des Steck- oder Buchsenkontakts des Koaxialsteckers 20, einen zweiten hohlzylindrischen Abschnitt 33 zur Aufnahme des zweiten und dritten Abschnitts 22, 23 des Koaxialsteckers 20 mit einem im Bereich des dritten zylindrischen Abschnitts 23 des Koaxialsteckers 20 liegenden Kontaktbelag 34 sowie einen dritten hohlzylindrischen Abschnitt 35 zur Aufnahme des vorderen Teils des vierten zylindrischen Abschnitts 24 des Koaxialsteckers 20 auf. Der leitende Kontaktbelag 34 ist vorzugsweise als federnder, leitender Belag ausgebildet, der einen festen, dauerhaften elektrischen Kontakt mit dem leitenden Belag des dritten zylindrischen Abschnitts 23 des Koaxialsteckers 20 bildet.

Im ersten und dritten hohlzylindrischen Abschnitt 32, 35 sind Dichtnuten 36, 37 vorgesehen, die mit den Dichtringen 26, 27 im ersten und vierten zylindrischen Abschnitt 21, 24 des Koaxialsteckers 20 im vollständig eingesteckten Zustand des Koaxialsteckers 20 eine körperflüssigkeitsdichte Verbindung eingehen. Wahlweise können die Dichtringe 26, 27 des Koaxialsteckers 20 oder die Dichtnuten 36, 37 elastisch ausgeführt sein, so daß eine elastisch federnde Verbindung gewährleistet ist.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Koaxialstecker zur Verbindung eines implantierbaren, bipolaren Herzschrittmachers mit einer Anode (2) und einer Kathode (8), die über je einen Anoden- (1) und Kathodenleiter (8) mit dem Koaxialstecker verbunden sind, wobei der Koaxialstecker jeweils eine mit dem Kathodenleiter (8) und dem Anodenleiter (1) elektrisch leitend verbundene Kontaktfläche und zwei axiale Bohrungen zur Aufnahme des Anoden- (1) und Kathodenleiters (8) aufweist,
**gekennzeichnet durch**
einen ersten zylindrischen Abschnitt (21), der die mit dem Kathodenleiter (8) elektrisch leitend verbundene Kontaktfläche aufweist,
einen sich an den ersten zylindrischen Abschnitt (21) anschließenden zweiten zylindrischen Abschnitt (22), der mit einer Ausnehmung (25) versehen ist, und
einen an den zweiten zylindrischen Abschnitt (22) anschließenden dritten zylindrischen Abschnitt (23), der die mit dem Anodenleiter (1) leitend verbundene Kontaktfläche aufweist,
durchgehende Bohrungen für den Anoden- (1) und den Kathodenleiter (9), in die ein Mandrin (10) zum jeweiligen Plazieren der Anode (2) und der Kathode (9) einführbar ist,
und eine stirnseitige, im Bereich der Ausnehmung (25) des zweiten zylindrischen Abschnitts (22) angeordnete, axiale Mandrinbohrung (29) zur Aufnahme des Mandrins (10) zum Placieren der Anode (2).

2. Koaxialstecker nach Anspruch 1, **dadurch gekennzeichnet,** daß die Ausnehmung (25) im zweiten zylindrischen Abschnitt (22) aus einer schräg verlaufenden Fläche besteht, die mit der die Mandrinbohrung (29) enthaltenden Stirnseite des dritten zylindrischen Abschnitts (23) einen stumpfen Winkel bildet.

3. Koaxialstecker nach Anspruch 1 oder 2, **dadurch gekennzeichnet,** daß ein vierter zylindrischer Abschnitt (24) vorgesehen ist, der den Steckergriff des Koaxialsteckers (20) bildet und eine erste, axial durchgehende und mit der Mandrinbohrung (29) fluchtende Bohrung (40) zur Aufnahme des Anodenleiters (1) und eine zweite, axial durchgehende und mit entsprechenden Bohrungen im ersten, zweiten und dritten zylindrischen Abschnitt (21, 22, 23) fluchtende Bohrung (41) zur Aufnahme des Kathodenleiters (8) aufweist, und daß die erste und zweite axial durchgehende Bohrung (40, 41) im vierten zylindrischen Abschnitt (24) und die mit ihnen fluchtenden Bohrungen (29; 42, 43) schräg von der Spitze des Koaxialsteckers (20) bzw. der Mandrinbohrung (29) zur Stirnseite (28) des vierten zylindrischen Abschnitts (24) verlaufen, wo der Anoden- und Kathodenleiter (1; 8) etwa mittig austreten.

4. Koaxialstecker nach einem der vorangehenden Ansprüche, **dadurch gekennzeichnet,** daß im Bereich des ersten und vierten Abschnitts (21, 24) des Koaxialsteckers (20) mindestens ein Dicht- und Isolationsring (26, 27) vorgesehen ist.

5. Koaxialstecker nach einem der vorangehenden Ansprüche, **dadurch gekennzeichnet,** daß der erste Abschnitt (21) einen mit dem Kathodenleiter (8) elektrisch leitend verbundenen, koaxial angeordneten Steckkontakt aufweist.

6. Koaxialstecker nach einem der vorangehenden Ansprüche, **dadurch gekennzeichnet,** daß der erste Abschnitt (21) eine mit dem Kathodenleiter (8) elektrisch leitend verbundene, koaxial angeordnete Steckbuchse aufweist.

7. Koaxialstecker nach einem der vorangehenden Ansprüche, **dadurch gekennzeichnet,** daß die mit dem Anodenleiter (1) verbundene Kontaktfläche des dritten zylindrischen Abschnitts (23) bis zum zweiten zylindrischen Abschnitt (22) verläuft.

## Claims

1. A coaxial connector for connecting an implantable, bipolar heart pacemaker to an anode (2) and a cathode (8) which are connected via in each case an anode (1) and cathode (8) conductor to the coaxial connector, in which the coaxial connector in each case has a contact surface electrically conductively connected to the cathode conductor (8) and the anode conductor (1) and has two axial bores for receiving the anode conductor (1) and the cathode conductor (8),
**characterised by**
a first cylindrical portion (21) which has the contact surface electrically conductively connected to the cathode conductor (8),
a second cylindrical portion (22) following on from the first cylindrical portion (21), which is provided with a recess (25) and
a third cylindrical portion (23) following on from the second cylindrical portion (22), which has the contact surface conductively connected to the anode conductor (1),
continuous bores for the anode conductor (1) and the cathode conductor (9), into which a mandrin (10) may be inserted for respectively placing the anode (2) and the cathode (9),
and an axial mandrin bore (29) for receiving the mandrin (10) which bore is arranged at the end in the region of the recess (25) of the second cylindrical portion (22), for placing the anode (2).

2. A coaxial connector according to claim 1, **characterised in that** the recess (25) in the second cylindrical portion (22) consists of an inclined surface which forms an obtuse angle with the end face of the third cylindrical portion (23), which contains the mandrin bore (29).

3. A coaxial connector accordig to claim 1 or 2, **characterised in that** a fourth cylindrical portion (24) is provided which forms the handle of the coaxial connector (20) and has a first, axially continuous bore in alignment with the mandrin bore (29), for receiving the anode conductor (1), and a second axially continuous bore (41) in alignment with corresponding bores in the first, second and third cylindrical portion (21, 22, 23) for receiving the cathode conductor (8), and that the first and second axially continuous bores (40, 41) in the fourth cylindrical portion (24) and the bores in alignment with them (29; 42,43) run inclined from the tip of the coaxial connector (20) and/or the mandrin bore (29) to the end face (28) of the fourth cylindrical portion (24) where the anode and cathode conductors (1;8) come out approximately in the middle.

4. A coaxial connector according to any one of the preceding claims, **characterised in that** in the region of the first and fourth portion (21, 24) of the coaxial connector (20) at least one sealing and insulation ring (26, 27) is provided.

5. A coaxial connector according to any one of the preceding claims **characterised in that** the first portion (21) has a coaxially arranged plug contact electrically conductively connected to the cathode conductor (8).

6. A coaxial connector according to any one of the preceding claims **characterised in that** the first portion (21) has a coaxially arranged connector socket electrically conductively connected to the cathode conductor (8).

7. A coaxial connector according to any one of the preceding claims **characterised in that** the contact surface of the third cylindrical portion (23) which is connected to the anode conductor (1) runs up to the second cylindrical portion (22).

## Revendications

1. Prise coaxiale pour la connexion d'un stimulateur cardiaque bipolaire implantable avec une anode (2) et une cathode (9) qui sont reliées à la prise coaxiale par un conducteur d'anode (1) et un conducteur de cathode (8), la prise coaxiale présentant une surface de contact reliée de manière électriquement conductrice au conducteur de cathode (8) et une surface de contact reliée de manière électriquement conductrice au conducteur d'anode (1) et deux alésages axiaux destinés à recevoir le conducteur d'anode (1) et le conducteur de cathode (8), caractérisée par
une première section cylindrique (21) qui comporte la surface de contact reliée de manière électriquement conductrice au conducteur de cathode (8),
une seconde section cylindrique (22) qui fait suite à la première section cylindrique (21) et qui est munie d'un évidement (25), et
une troisième section cylindrique (23) qui fait suite à la seconde section cylindrique (22) et qui comporte la surface de contact reliée de manière électriquement conductrice au conducteur d'anode (1),
des alésages traversants pour le conducteur d'anode (1) et le conducteur de cathode (8), dans lesquels un mandrin (10) destiné au positionnement de l'anode (2) et de la cathode (9) peut être introduit,
et un alésage de mandrin (29) axial, frontal, agencé dans le domaine de l'évidement (25) de la seconde section cylindrique (22) et destiné à recevoir le mandrin (10) pour le positionnement de l'anode (2).

2. Prise coaxiale selon la revendication 1, caractérisée en ce que l'évidement (25) de la seconde section cylindrique (22) consiste en une surface qui s'étend obliquement et qui forme un angle obtu avec le côté frontal de la troisième section cylindrique (23) qui contient l'alésage de mandrin (29).

3. Prise coaxiale selon la revendication 1 ou 2, caractérisée en ce qu'il est prévu une quatrième section cylindrique (24) qui forme la poignée de la prise coaxiale (20) et qui présente un premier alésage (40) axialement traversant, aligné avec l'alésage de mandrin (29) et destiné à recevoir le conducteur d'anode (1) et un second alésage (41) axialement traversant, aligné avec des alésage correspondants dans les première, seconde et troisième sections cylindriques (21, 22, 23) et destiné à recevoir le conducteur de cathode (8), et en ce que les premier et second alésages axialement traversants (40, 41) de la quatrième section cylindrique (24) et les alésages (29 ; 42, 43) alignés avec les précédents s'étendent en biais de la pointe de la prise coaxiale (20) ou de l'alésage de mandrin (29) au côté frontal (28) de la quatrième section cylindrique (24) où les conducteurs d'anode et de cathode (1 ; 8) sortent de manière sensiblement centrée.

4. Prise coaxiale selon l'une des revendications précédentes, caractérisée en ce qu'il est prévu au moins une bague d'étanchéité et d'isolation (26, 27) dans le domaine des première et quatrième sections (21, 24) de la prise coaxiale (20).

5. Prise coaxiale selon l'une des revendications précédentes, caractérisée en ce que la première section (21) comporte une fiche mâle coaxiale, reliée de manière électriquement conductrice au conducteur de cathode (8).

6. Prise coaxiale selon l'une des revendications précédentes, caractérisée en ce que la première section (21) comporte une douille coaxiale, reliée de manière électriquement conductrice au conducteur de cathode (8).

7. Prise coaxiale selon l'une des revendications précédentes, caractérisée en ce que la surface de contact de la troisième section cylindrique (23) qui est reliée au conducteur d'anode (1) s'étend jusqu'à la seconde section cylindrique (22).
